Europäisches Patentamt

European Patent Office

Office européen des brevets

(11) Veröffentlichungsnummer: **0 176 698**
**B1**

(12) **EUROPÄISCHE PATENTSCHRIFT**

(45) Veröffentlichungstag der Patentschrift:
16.09.87

(51) Int. Cl.⁴: **C 07 C 59/245,** C 07 C 51/42,
**C 12 P 7/46**

(21) Anmeldenummer: **85109749.3**

(22) Anmeldetag: **02.08.85**

(54) Verfahren zur Gewinnung von L-Äpfelsäure.

(30) Priorität: **22.09.84 DE 3434918**

(43) Veröffentlichungstag der Anmeldung:
**09.04.86 Patentblatt 86/15**

(45) Bekanntmachung des Hinweises auf die Patenterteilung:
**16.09.87 Patentblatt 87/38**

(84) Benannte Vertragsstaaten:
**AT BE CH DE FR GB IT LI NL SE**

(56) Entgegenhaltungen:
**EP - A - 0 115 606**
**DE - A - 1 417 033**
**DE - A - 1 543 977**

**CHEMICAL ABSTRACTS, Band 80, Nr. 9, 4. März 1974,
Columbus, Ohio, USA, G.G. BABAYAN et al.
"Purification of alpha-tartaric acid and its alkali metal
salts of microimpurities", Seite 318, Zusammenfassung
Nr. 47 469b**

(73) Patentinhaber: **HÜLS AKTIENGESELLSCHAFT, - RSP
Patente / PB 15 - Postfach 13 20, D-4370 Marl 1 (DE)**

(72) Erfinder: **Seipenbusch, Reinhold, Dr.,
Paul-Löbe-Strasse 62, D-6390 Gladbeck (DE)**

# Beschreibung

Die Erfindung betrifft ein Verfahren zur Gewinnung von L-Äpfelsäure (LÄS) durch biotechnische Umsetzung von Fumarsäure. Dabei entsteht ein Reaktionsgemisch, in dem neben der gewünschten LÄS nicht umgesetzte Fumarsäure und andere nichtionogene Stoffe aus der biotechnischen Umsetzung enthalten sind. Die LÄS und die Fumarsäure liegen in der Regel als Salze vor.

Die Erfindung bezweckt, das Abtrennen der LÄS aus dem Reaktionsgemisch wirtschaftlicher zu gestalten. Die LÄS soll in möglichst reiner Form gewonnen werden, und andere im Reaktionsgemisch enthaltene Stoffe sollen in wiederverwendbarer Form anfallen.

Die biotechnische Umsetzung von Fumarsäure in LÄS ist bekannt und wird nach unterschiedlichen Verfahren durchgeführt. Es können Bakterien (DE-AS 23 63 285), immobilisierte Bakterien (Europ. J. Appl. Microbiol. *3* (1976), Seiten 169 bis 183; US-3.922.195), Pilze (DE-OS 33 10 849), immobilisierte Pilze (DE-OS 24 50 137) oder das aus Mikroorganismen isolierte Enzym Fumarase (DE-AS 24 15 310) zu dieser biotechnischen Umsetzung benutzt werden. Das LÄS-Salz kann in dem Reaktionsgemisch in mässig hoher Konzentration oder in hoher Konzentration vorliegen (DE-OS 33 10 849 und DE-OS 34 34 880). Weiter ist die biotechnische Umsetzung von Glucose in LÄS durch Vergesellschaftung eines Pilzes mit einem Bakterium beschrieben worden (J. Ferment. Technol. *54* (1976), Seiten 197 bis 204).

Zur Abtrennung der LÄS aus dem Reaktionsgemisch sind verschiedene Verfahren bekannt. Dem Fermentationsansatz wird beispielsweise eine grosse Menge einer Kalzium-Verbindung zugegeben, wodurch die gebildete LÄS bereits während der Fermentation als Ca-Malat ausfällt (DE-OS 14 17 033). Das Reaktionsgemisch kann auch mit Kationenaustauschern behandelt werden; die Eluate werden nach Konzentrierung filtriert und eingedampft (DE-OS 32 47 981). Weiter kann man aus dem Reaktionsgemisch zunächst die nicht umgesetzte Fumarsäure durch Ansäuern abscheiden und danach die LÄS als Kalzium-Salz fällen. Dieses Salz kann mit Schwefelsäure zu Kalziumsulfat und LÄS umgesetzt werden, die zum Entfernen des gelösten Kalziumsulfats in einem Ionenaustauscher behandelt wird.

Die bekannten Verfahren zur Gewinnung von LÄS durch biotechnische Umsetzung von Fumarsäure haben unter anderem die folgenden Eigenschaften:

— Beim Ansäuern des Reaktionsgemisches zwecks Abscheiden der nicht umgesetzten Fumarsäure bildet sich aus der zugegebenen Säure und den im Reaktionsgemisch enthaltenen Kationen ein Salz, das nach der anschliessenden Kalzium-Fällung der LÄS zwar zum grössten Teil im Filtrat bleibt, jedoch zum gewissen Teil im Filterkuchen aus Ca-Malat enthalten ist.

— Der Filterkuchen muss salzfrei und sauber gewaschen werden. Dabei treten Verluste an Ca-Malat auf, wodurch die Ausbeute an LÄS gemindert wird. Filtrat und Waschwasser werden verworfen.

— Die vor der biotechnischen Umsetzung zum Neutralisieren der Fumarsäure zugesetzte Lauge kann praktisch gar nicht zurückgewonnen werden und ist damit nach einmaligen Gebrauch vollständig verloren.

— Beim Umsetzen des Ca-Malats mit Schwefelsäure entsteht Ca-Sulfat (Gips) als unvermeidliches Nebenprodukt. Dessen Beseitigung belastet das Verfahren mit zusätzlichen Kosten.

— Es ist oft schwierig, die LÄS in der gewünschten hohen Reinheit bei gleichzeitig hoher Ausbeute zu erhalten.

Damit stellt sich die Aufgabe, die Abtrennung der LÄS aus dem Reaktionsgemisch zu verbessern, damit man die LÄS in hoher Reinheit und mit grosser Ausbeute unter wirtschaftlichen Bedingungen erhält.

Diese Aufgabe wird erfindungsgemäss gelöst durch ein Verfahren zur Gewinnung von LÄS aus den bei der biotechnischen Umsetzung von Fumarsäure-Salzen zu LÄS-Salzen anfallenden Reaktionsgemischen, die praktisch frei von Zellmasse sind oder davon vorher befreit worden sind, das folgende kennzeichnende Merkmale hat:

— kontinuierliches Trennen des Reaktionsgemisches in einer bekannten Elektro-Dialyse-Einheit (EDE) mit mindestens drei Zellen in

— das Säurekonzentrat, das LÄS und Fumarsäure enthält,

— das Laugekonzentrat,

— das Diluat, das die nichtionogenen Bestandteile des Reaktionsgemisches und Reste von Fumarat und L-Malat enthält,

— Aufarbeiten des Säurekonzentrats auf reine LÄS nach bekannten Verfahren und

— Sammeln des Laugekonzentrats für dessen Wiederverwendung.

Das Laugekonzentrat besteht aus gelösten Hydroxiden der Elemente der ersten Gruppe des Periodensystems, bevorzugt Natrium und Kalium, und/oder des Ammoniaks. Das Laugekonzentrat kann in die biotechnische Umsetzung der Fumarsäure in LÄS zurückgeführt und dort erneut zur Neutralisation der Fumarsäure benutzt werden.

Das Säure- und das Laugekonzentrat werden kontinuierlich der EDE entnommen. Ausser den drei Zellen, die für den Säure-Kreislauf, den Lauge-Kreislauf und den Salz-/Diluat-Kreislauf benutzt werden, kann die EDE noch einen Anoden-Spülkreislauf und gegebenenfalls noch einen Kathoden-Spülkreislauf enthalten. Der Anoden-Spülkreislauf enthält eine Säurelösung, bevorzugt eine LÄS-Lösung. Der gegebenenfalls vorhandene Kathoden-Spülkreislauf enthält eine Laugelösung, und zwar bevorzugt die Lauge, die auch zum Neutralisieren der Fumarsäure vor Beginn der biotechnischen Umsetzung benutzt wurde.

Das Verfahren wird besonders wirtschaftlich, wenn man das Reaktionsgemisch in einer EDE trennt, die mindestens eine bipolare Membran enthält. An dieser Membran werden Wasserstoff- und Hydroxyl-Ionen durch elektrodialytische Dissoziation erzeugt, die unmittelbar zum Neutralisieren der Säure- und der Lauge-Reste benutzt werden können. Bei diesem Vorgang entsteht praktisch kein Wasserstoff- und kein Sauerstoff-Gas.

Weitere Einzelheiten zur Elektrodialyse sind beispielsweise angegeben in Chem. -Ing. -Techn. *56* (1984), Seiten 214 bis 220 und Kirk-Othmer, Encyl. Chem. Technology, 3. Auflage, Band 8 (1979), Seiten 726 bis 738.

Sobald sich im Säure- und im Lauge-Kreislauf die gewünschten Konzentrationen eingestellt haben, werden ein Säure- und ein Lauge-Teilstrom kontinuierlich jedem Kreislauf entnommen. Die entnommenen Flüssigkeitsmengen werden kontinuierlich durch Wasser ersetzt. Der Austrag an Säure und an Lauge ist genau so gross wie der Durchsatz an Säure und an Lauge durch die Membranen der EDE.

Im Säurekonzentrat fällt die Fumarsäure in der Regel bereits zum grossen Teil aus; sie kann auf einem Filter im Säure-Kreislauf zurückgehalten und dem Kreislauf entnommen werden. Diese Fumarsäure wird wieder in den Bio-Reaktor zur biotechnischen Umsetzung in LÄS gegeben.

Zu Beginn der Elektrodialyse wird der Salz-/Diluat-Kreislauf mit dem Reaktionsgemisch aus der biotechnischen Umsetzung von Fumarat in L-Malat beschickt. Mit dem Fortschreiten der Elektrodialyse verarmt das Reaktionsgemisch an Fumarat und L-Malat, bis es nur noch Reste dieser Salze enthält und als Diluat vorliegt. Dann werden die Elektrodialyse und der Diluat-Kreislauf unterbrochen, das gesamte Diluat wird entnommen. Gegebenenfalls wird dieser Kreislauf mit Wasser gespült. Dem Spülwasser kann Ammoniak oder ein Fungizid/Bakterizid zugesetzt werden, damit die Mikroorganismen, die im Kreislauf (z. B. an den Membranen) hängengeblieben sind, abgetötet werden.

Anschliessend wird der Salz-/Diluat-Kreislauf mit frischem Reaktionsgemisch gefüllt, und die Elektrodialyse wird erneut gestartet.

Das Säurekonzentrat wird im ersten Konzentrierungsschritt bei einer Temperatur unterhalb 60° C auf eine Konzentration der LÄS von 70 bis 75 Gewichtsprozent gebracht und auf Umgebungstemperatur abgekühlt. Die dabei auskristallisierende Fumarsäure, die nur wenig LÄS enthält, wird abgetrennt. Die verbliebene LÄS-Lösung wird im zweiten Konzentrierungsschritt bei einer Temperatur unterhalb 60° C auf eine Konzentration der LÄS von über 80 Gewichtsprozent gebracht und auf Umgebungstemperatur abgekühlt. Die dabei auskristallisierte LÄS, die den grössten Teil der restlichen Fumarsäure enthält, wird abgetrennt. Bei erhöhten Reinheitsforderungen kann dieser zweite Konzentrierungsschritt wiederholt werden. Die nach dem zweiten Konzentrierungsschritt verbliebene LÄS-Lösung wird — bevorzugt bei Unterdruck — stufenweise eingeengt, bis die Flüssigkeitsmenge nur noch ca. 10% der Ausgangsmenge für die letzte Konzentrierung beträgt.

Die beim Abkühlen nach dem ersten Konzentrierungsschritt auskristallisierte Fumarsäure, die nur wenig LÄS enthält, wird in den Bio-Reaktor zur Gewinnung von LÄS aus biotechnischer Umsetzung zurückgeführt.

Die beim Abkühlen nach dem zweiten Konzentrierungsschritt auskristallisierte Fumarsäurehaltige LÄS wird in das für den ersten Konzentrierungsschritt bestimmte Säurekonzentrat zurückgeführt.

Die beim letzten Konzentrierungsschritt verbliebene Flüssigkeit ist eine gesättigte LÄS-Lösung und enthält praktisch das gesamte L-Malat und Fumarat in gelöster Form. Diese Flüssigkeit wird bevorzugt über einen stark sauren Kationenaustauscher gefahren zwecks Spaltung von Malat und Fumarat. Danach wird die Flüssigkeit dem Säurekonzentrat wieder zugegeben.

Bei geringeren Forderungen an die Reinheit der LÄS kann gegebenenfalls einer oder mehrere der beschriebenen Aufarbeitungsschritte entfallen.

Das erfindungsgemässe Verfahren hat folgende Vorteile:
— die LÄS und die Fumarsäure werden ohne Fällungsreaktionen direkt abgeschieden.
— Es geht praktisch keine Fumarsäure verloren.
— Die Wertstoffe werden schonend gewonnen.
— Das Reaktionsgemisch kann in der EDE kontinuierlich zu den gewünschten Säure- und Lauge-Konzentraten aufgearbeitet werden.

Ein für das erfindungsgemässe Verfahren geeignetes Reaktionsgemisch wird beispielsweise wie folgt hergestellt. Die in einem bekannten Bio-Reaktor unter sterilen Bedingungen gezüchtete Zellmasse (z. B. des Pilzes *Aspergillus wentii*) wird aus der Kulturflüssigkeit abgetrennt und als Inoculum einem einfach gebauten Behälter zugeführt, der eine (Ammonium-)Fumarat-Lösung enthält. Diese Lösung wird gerührt und mit der für die Erhaltungsatmung des Pilzes notwendigen geringen Sauerstoffmenge versetzt. Die biotechnische Umsetzung ist beendet, sobald ca. 85 Gewichtsprozent des Fumarats als L-Malat vorliegen.

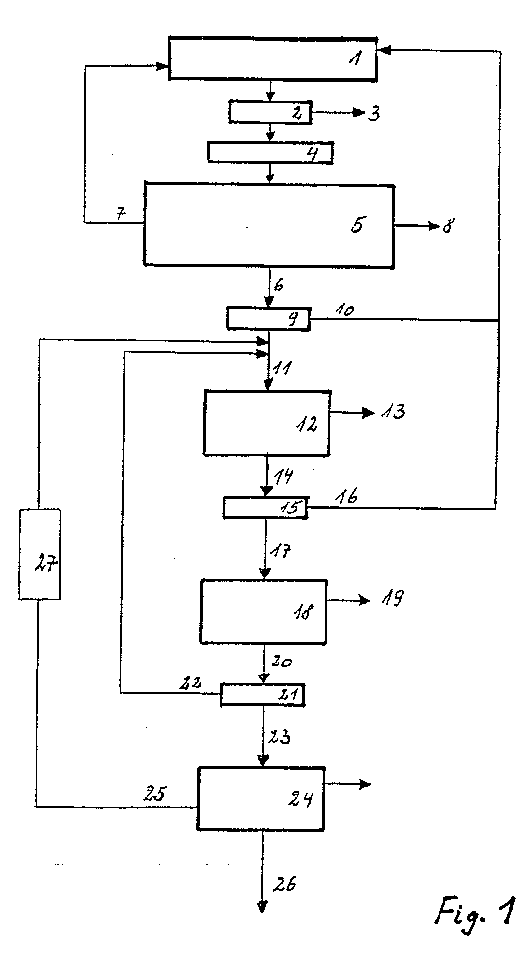

Aus diesem Reaktionsgemisch wird die Zellmasse nach bekannten Methoden abgetrennt. Das Reaktionsgemisch wird — gegebenenfalls nach Filtrieren über Aktivkohle — in der EDE aufgetrennt. Das Säurekonzentrat wird aufgearbeitet, wie in Figur 1 schematisch dargestellt ist. Das Laugekonzentrat wird in den Bio-Reaktor zurückgeführt, das praktisch salzfreie Diluat wird verworfen.

Das Fliessbild beginnt mit dem Reaktor (1) zur biotechnischen Umsetzung von Fumarat in L-Malat. Das Reaktionsgemisch wird dem Reaktor entnommen, und mittels des Abscheiders (2) wird die Zellmasse (3) daraus abgetrennt. Das Reaktionsgemisch wird gegebenenfalls über eine Aktivkohle-Filter (4) gegeben und der EDE (5) vorgelegt. Die Kreisläufe durch die EDE sind nicht eingezeichnet. Aus der EDE werden Säurekonzentrat (6) und Laugekonzentrat (7) kontinuierlich abgezogen. Beide Konzentrate können zunächst in nicht gezeichneten Behältern gesammelt werden, bevor das Laugekonzentrat in den Reaktor (1) und das Säurekonzentrat der Aufarbeitung zugeführt werden.

Sobald die Salzkonzentration im Salz-/Diluat-Kreislauf auf den vorgegebenen Wert gesunken ist, wird das Diluat (8) vollständig aus dem Kreislauf entfernt. Dieser Kreislauf kann mit Wasser gespült werden, bevor er mit einer neuen Menge Reaktionsgemisch beschickt wird.

Das Säurekonzentrat (6) wird durch einen Abscheider (9) geleitet, um auskristallisierte Fumarsäure abzuscheiden. Die Fumarsäure kann bereits im Säurekonzentrat auskristallisieren, falls dort ihre Sättigungskonzentration überschritten wird. Wahlweise kann dieser Abscheider auch im Säurekreislauf angeordnet werden. Die Fumarsäure (10) wird — gegebenenfalls nach Zwischenlagerung — in den Reaktor zur biotechnischen Umsetzung zurückgeführt.

Das Säurekonzentrat (11) wird im (Vakuum-) Verdampfer (12) im ersten Konzentrierungsschritt aufkonzentriert, wobei Wasser (13) freigesetzt wird. Aus den abgekühlten Säurekonzentrat (14) wird im Abscheider (15) weitere Fumarsäure (16) abgetrennt und ebenfalls — eventuell nach Zwischenlagerung — in den Reaktor (1) zurückgeführt. Das Säurekonzentrat (17) wird im Vorkristallisator (18) (z. B. Verdampfungskristallisator) im zweiten Konzentrierungsschritt aufkonzentriert, wobei weiteres Wasser (19) freigesetzt wird. Aus dem abgekühlten Säurekonzentrat (20) wird im Abscheider (21) Fumarsäure-haltige LÄS (22) abgeschieden und vor den Verdampfer (12) zurückgeführt. Die konzentrierte LÄS-Lösung (23) wird im Verdampfungskristallisator und Abscheider (24) z. B. stufenweise eingeengt bis auf einen Rest (25) der vorgelegten Flüssigkeit.

Die auskristallisierte LÄS (26) wird abgeschieden und der Vorrichtung entnommen. Die restliche Flüssigkeit (25) wird nach Umsetzen des nicht gespaltenen Malats und Fumarats — bevorzugt über Kationenaustauscher (27) — dem Säurekonzentrat (11) vor dem Verdampfer (12) beigemischt.

Bei erhöhten Reinheitsforderungen wird die LÄS-Lösung (23) gegebenenfalls nochmals durch einen Vorkristallisator (18) und Abscheider (21) gefahren, wobei erneut Wasser (19) und Fumarsäure-haltige LÄS (22) abgeschieden wird.

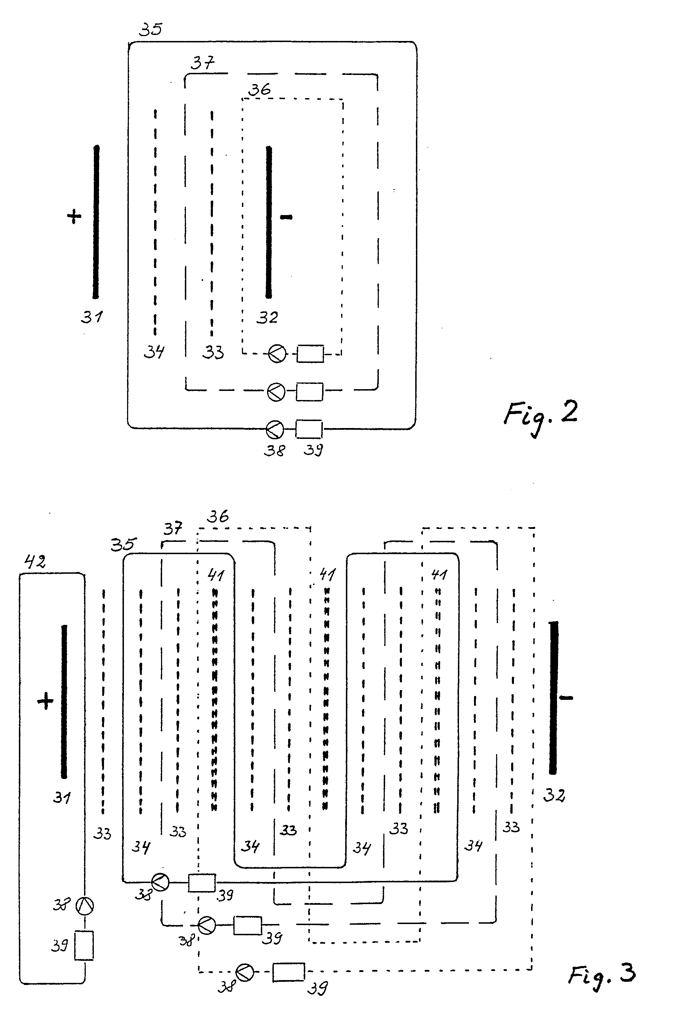

Figur 2 zeigt schematisch die Kreisläufe durch eine dreizellige EDE. Zwischen der Anode (31) und der Kathode (32) sind eine Kationenaustauscher-Membran (33) und eine Anionenaustauscher-Membran (34) angeordnet. Der Säure-Kreislauf (35) geht durch die vor der Anode liegenden Zelle, der Lauge-Kreislauf (36) geht durch die vor der Kathode liegenden Zelle. Der Salz-/Diluat-Kreislauf (37) geht durch die mittlere Zelle, die anodenseitig von der Anionenaustauscher-Membran und kathodenseitig von der Kationenaustauscher-Membran begrenzt wird. Die Flüssigkeiten werden jeweils durch Pumpen (38) umgewälzt. Jeder Kreislauf enthält jeweils eine Pumpenvorlage (39). Die weiteren Hilfsvorrichtungen und die Stromversorgung für die EDE sind nicht eingezeichnet.

Figur 3 zeigt schematisch die Kreisläufe durch eine EDE, die drei bipolare Membranen (41) enthält. Die Kreisläufe sind analog zu Figur 2. Ausserdem enthält diese EDE einen Anoden-Spülkreislauf (42).

Die Zuleitungen und Ableitungen für die Kreisläufe sind in Figur 2 und Figur 3 nicht eingezeichnet.

Das erfindungsgemässe Verfahren wird anhand des folgenden Beispiels weiter erläutert, ohne hierauf beschränkt zu sein.

*Beispiel*

Die EDE besteht aus drei Zellen und enthält eine Anionenaustauscher-Membran und eine Kationenaustauscher-Membran, die analog zu Figur 2 angeordnet sind. Die handelsüblichen Membranen bestehen beispielsweise aus vernetztem Polystyrol. Die Kationenaustauscher-Membran enthält durch Sulfonierung eingebrachte $SO_3^-$-Gruppen, die Anionenaustauscher-Membran quarternäre Ammoniumgruppen ($NH_4^+$).

Jede Membran hat eine aktive Fläche von 148 cm². Der Abstand der Membranen voneinander und von den Elektroden beträgt jeweils ca. 1,4 cm.

Vor Beginn der Elektrodialyse enthalten die Kreisläufe folgende Lösungen:
— Salz-/Diluat-Kreislauf: 350 g einer wässerigen Lösung, die 144,1 g $NH_4$-Malat (ca. 41,2 Gewichtsprozent) und 16,3 g $NH_4$-Fumarat (ca. 4,7 Gewichtsprozent) enthält,
— Säure-Kreislauf: 400 g einer Lösung von 40 g LÄS in destilliertem Wasser,
— Lauge-Kreislauf: 500 g Trinkwasser mit einer kleinen Menge $NH_3$ zwecks Erhöhung der Leitfähigkeit.

Während der Elektrodialyse fliesst durch die EDE ein Gleichstrom von ca. 1,5 A. Die Gleichspannung beträgt zu Beginn der Elektrodialyse ca. 10 V, gegen Ende ca. 6 V; sie wird auf konstanten Strom durch die EDE geregelt.

Das an der Kathode entstehende $NH_3$-Gas wird zurückgewaschen. Die Umwälzpumpen fördern jeweils ca. 10 Liter pro Stunde. Die Zellen sind dauernd vollständig mit Flüssigkeit gefüllt. Für dieses Beispiel wurde den Kreisläufen weder Flüssigkeit entnommen noch zugeführt.

Nach 36 Betriebsstunden wurden folgende Ergebnisse erhalten:

| | Diluat (g) | Säure-Konzentrat (g) | Lauge-Konzentrat (g) |
|---|---|---|---|
| LÄS | — | 146,0 | — |
| Fumarsäure | — | 12,3 | — |
| $(NH_4)_2$-Malat | 7,5 | 1,8 | 1,9 |
| $(NH_4)_2$-Fumarat | 0,1 | 0,1 | 0,1 |
| $NH_3$ | — | — | 30,5 |

Die Fumarsäure lag im Säurekonzentrat weitgehend in kristallisierter Form vor. Aus dem Säurekonzentrat wurden 8,2 g Kristallisat abgetrennt, das praktisch ganz aus Fumarsäure bestand.

Das Säurekonzentrat wurde bei 50° C und einem Druck von ca. 50 mbar (absolut) zum ersten Mal auf einen Gehalt von 73 Gewichtsprozent LÄS konzentriert und auf 23° C abgekühlt. Es wurden 4,7 g Kristallisat abgetrennt, das aus 1,2 g LÄS und 3,5 g Fumarsäure bestand.

Das verbliebene Säurekonzentrat wurde bei ca. 50° C und einem Druck von ca. 50 mbar (absolut) zum zweiten Mal auf einen Gehalt von 83 Gewichtsprozent LÄS konzentriert und auf 23° C abgekühlt. Es wurden 36,2 g feuchtes Kristallisat abgetrennt, das aus 35,0 g LÄS und 0,5 g Fumarsäure bestand.

Das verbliebene Säurekonzentrat wurde bei ca. 50° C und einem Druck von ca. 40 mbar (absolut) stufenweise eingeengt bis auf eine restliche Flüssigkeitsmenge. Es wurden 108,3 g Kristallisat und 11,6 g Flüssigkeit erhalten.

Das Kristallisat bestand aus
97,7 Gewichtsprozent LÄS
0,1 Gewichtsprozent Fumarsäure
1,9 Gewichtsprozent Wasser
< 0,1 Gewichtsprozent Sulfatasche

Das Kristallisat wurde auf einen Wassergehalt von unter 1% getrocknet.

Das hier beschriebene Verfahren kann auch kontinuierlich durchgeführt werden.

## Patentansprüche

1. Verfahren zur Gewinnung von L-Äpfelsäure aus den bei der biotechnischen Umsetzung von Fumarsäure-Salzen zu L-Äpfelsäure-Salzen anfallenden Reaktionsgemischen, die praktisch frei von Zellmasse sind oder davon vorher befreit worden sind, gekennzeichnet durch
— kontinuierliches Trennen des Reaktionsgemisches in einer bekannten Elektro-Dialyse-Einheit mit mindestens drei Zellen in
— das Säurekonzentrat, enthaltend L-Äpfelsäure und Fumarsäure,
— das Laugekonzentrat,
— das Diluat, enthaltend die nichtionogenen Bestandteile des Reaktionsgemisches und Rest von Fumarat und L-Malat und andere Salze,
— Aufarbeiten des Säurekonzentrats auf reine L-Äpfelsäure nach bekannten Verfahren,
— Sammeln des Laugekonzentrats für dessen Wiederverwendung.

2. Verfahren nach Anspruch 1, gekennzeichnet durch
— Rückführen des Laugekonzentrats in die biotechnische Umsetzung der Fumarsäure in L-Äpfelsäure.

3. Verfahren nach den Ansprüchen 1 und 2, gekennzeichnet durch
— kontinuierliches Trennen des Reaktionsgemisches in einer Elektro-Dialyse-Einheit, in der die Wasserstoff- und Hydroxylionen, die zum Neutralisieren der Säurereste bzw. der Basereste benötigt werden, vorwiegend innerhalb der Elektro-Dialyse-Einheit an mindestens einer bipolaren Membran erzeugt werden.

4. Verfahren nach den Ansprüchen 1 bis 3, gekennzeichnet durch folgende Aufarbeitungsstufen des Säurekonzentrats:
— erstes Konzentrieren des Säurekonzentrats bei einer Temperatur unterhalb 60° C auf eine Konzentration der L-Äpfelsäure von 70 bis 75 Gewichtsprozent, Abkühlen auf Umgebungstemperatur und Abtrennen der auskristallisierten Fumarsäure, die nur wenig L-Äpfelsäure enthält,
— zweites Konzentrieren der verbliebenen L-Äpfelsäure-Lösung bei einer Temperatur unterhalb 60° C auf eine Konzentration der L-Äpfelsäure von über 80 Gewichtsprozent, Abkühlen auf Umgebungstemperatur und Abtrennen der auskristallisierten L-Äpfelsäure, die Reste der Fumarsäure enthält,
— Eindampfen der L-Äpfelsäure-Lösung (bevorzugt bei Unterdruck) bis auf ca. 10% der in diesem Schritt vorgelegten Flüssigkeitsmenge,
— Abtrennen der auskristallisierten reinen L-Äpfelsäure.

5. Verfahren nach Anspruch 4, gekennzeichnet durch
— Rückführen der beim Abkühlen nach dem ersten Konzentrieren auskristallisierten Fumarsäure, die nur wenig L-Äpfelsäure enthält, in den Bio-Reaktor zur biotechnischen Umsetzung in L-Äpfelsäure.

6. Verfahren nach den Ansprüchen 4 und 5, gekennzeichnet durch
— Rückführen der beim Abkühlen nach dem zweiten Konzentrieren auskristallisierten Fumarsäurehaltigen L-Äpfelsäure in das für das erste konzentrieren bestimmte Säurekonzentrat.

## Claims

1. A process for preparing L-malic acid by separation from a reaction mixture produced in the biotechnical conversion of fumaric acid salts to L-malic acid salts, which mixture is almost free of cellular substances or which has been separated from them, comprising
— continuously separating the reaction mixture in a known electrodialysis unit having at least three cells in three solutions, which are
— the acid concentrate solution containing L-malic acid and fumaric acid,
— the alkali concentrate solution,
— the diluted product solution containing nonionic components of the reaction mixture and residues of fumaric acid salt and L-malic acid salt and other salts,
— working-up the acid concentrate solution by known methods to separate out pure L-malic acid,
— collecting the alkali concentrate solution for the reuse of this solution.

2. A process of claim 1, comprising
— recycling said alkali concentrate solution into the biotechnical conversion of fumaric acid to L-malic acid.

3. A process of claims 1 and 2, comprising
— continuously separating the reaction mixture in an electrodialysis unit within which the hydrogen ions and the hydroxyl ions required for neutralization of the acidic and alkaline species are produced preferably within the electrodialysis unit using at least one bipolar membrane.

4. A process of claims 1 to 3, comprising the following working-up steps for the acid concentrate:

— in a first concentration step, concentrating the acid concentrate solution at a temperature below 60° C to achieve a concentration of L-malic acid therein of 70 to 75% by weight, cooling the concentrate solution to ambient temperature, and separating the crystallized fumaric acid, which contains only a small amount of L-malic acid, from the resultant L-malic acid solution,

— in a second concentration step, concentrating the resultant L-malic acid solution at a temperature below 60° C to achieve a concentration of L-malic acid therein of more than 80% by weight, cooling the solution to ambient temperature, and separating the crystallized L-malic acid, which contains a residual amount of fumaric acid, from the remaining L-malic acid solution,

— evaporating the L-malic acid solution (preferably at reduced pressure) remaining after said second concentration step to about 10% of its weight at the end of the second concentration step,

— separating the resultant crystallized pure L-malic acid.

5. A process of claim 4, comprising
— recycling the fumaric acid crystallized-out in the cooling step after said first concentration step back into the bio-reactor for biotechnical conversion to L-malic acid.

6. A process of claims 4 and 5, comprising
— recycling the fumaric-acid-containing L-malic acid, crystallized-out in the cooling step after the second concentration step, back into the acid concentrate solution fed into the first concentration step.

## Revendications

1. Procédé pour l'obtention d'acide malique lévogyre à partir des mélanges réactionnels qui se forment lors de la conversion biotechnique de sels de l'acide fumarique en acide malique lévogyre et qui sont pratiquement exempts de masse cellulaire ou en ont été préalablement débarrassés, caractérisé par le fait que

— l'on sépare continuellement le mélange réactionnel, dans une unité d'électrodialyse connue comportant aux moins 3 cellules, en

— le concentrat d'acide renfermant de l'acide malique lévogyre et de l'acide fumarique,

— le concentrat de lessive,

— le diluat renfermant les constituants non ionogènes du mélange réactionnel et les restes de fumarate et de L-malate et d'autres sels,

— l'on traite le concentrat d'acide, suivant des procédés connus, pour obtenir l'acide malique lévogyre à l'état pur,

— que l'on rassemble le concentrat de lessive pour le réutiliser.

2. Procédé selon la revendication 1, caractérisé par le fait que

— l'on renvoie le concentrat de lessive dans l'étage biotechnique de transformation d'acide fumarique en acide malique lévogyre.

3. Procédé selon les revendications 1 et 2, caractérisé par le fait que

— l'on sépare en continu le mélange réactionnel dans une unité d'électrodialyse, dans laquelle les ions hydrogène et les ions hydroxyle, qui sont nécessaires pour neutraliser les restes d'acide et/ou les restes de base, sont produits principalement à l'intérieur de l'unité d'électrodialyse, sur au moins une membrane bipolaire.

4. Procédé selon les revendications 1 à 3, caractérisé par le fait que l'on effectue les stades suivants de traitement du concentrat d'acide:

— première concentration du concentrat d'acide à une température inférieure à 60° C jusqu'à atteindre une concentration en acide malique lévogyre de 70 à 75% en poids, refroidissement à la température ambiante et séparation de l'acide fumarique cristallisé, qui ne renferme que peu d'acide malique lévogyre,

— seconde concentration de la solution restante d'acide malique lévogyre à une température inférieure à 60° C jusqu'à atteindre une concentration d'acide malique lévogyre supérieure à 80% en poids, refroidissement à la température ambiante et séparation de l'acide malique lévogyre cristallisé, qui renferme des restes d'acide fumarique,

— concentration de la solution d'acide malique lévogyre (de préférence sous une dépression) jusqu'à avoir environ 10% de la quantité de liquide préalablement mise en place dans ce stade,

— séparation de l'acide malique lévogyre qui a cristallisé à l'état pur.

5. Procédé selon la revendication 4, caractérisé par le fait que

— l'on renvoie dans le bioréacteur, à la conversion biotechnique en acide malique lévogyre, l'acide fumarique ayant cristallisé par refroidissement après la première concentration et ne renfermant que peu d'acide malique lévogyre.

6. Procédé selon les revendications 4 et 5, caractérisé par le fait que

— l'on renvoie dans le concentrat d'acide destiné à la première concentration l'acide malique lévogyre renfermant de l'acide fumarique, qui a cristallisé par refroidissement après la seconde concentration.

Fig. 1

0 176 698

Fig. 2

Fig. 3

9